# EUROPEAN PATENT SPECIFICATION

(11) **EP 1 859 772 B1**
(45) Date of publication and mention of the grant of the patent: **15.12.2010**
(21) Application number: 06425351.1
(22) Date of filing: 24.05.2006
(51) Int. Cl.: A61J 1/10, B32B 27/08

(54) **Alkalized local anesthetic in bag**
Alkalisiertes Lokalanästhetikum im Beutel
Anesthésique local alcalinisé dans poche

(43) Date of publication of application: 28.11.2007
(73) Proprietor: Guardant S.r.l., 00044 Frascati RM (IT)
(72) Inventor: Pelloni, Vittorio, 00046 Grottaferrata (RM) (IT)
(74) Representative: Papa, Elisabetta

(56) References cited:
- EP-A2- 1 308 149
- GB-A- 1 059 554
- US-A- 4 340 049
- US-A- 4 623 334
- US-A- 5 409 125
- US-A- 5 896 989
- US-A- 6 068 617

## Description

The present invention refers to a pharmacological product comprising a fluid container receiving a local anesthetic.

For several years now, research by pharmaceutical industries aimed at a local anesthetic possessing an optimal therapeutic index, i.e. an effective dose/toxic dose ratio maximally reducing risks to the patient. In clinical practice, the effectiveness of a local anesthetic is assessed through the time of appearance of its effect (i.e., the so-called onset time or latency time) the duration of this effect and the quality of the sensory and motor block caused. Of course, besides from the physico-chemical qualities of the anesthetic, a motor and sensory block depends also from the volume and concentration of the administered drug. In general, by increasing the dosage the quality of the block improves yet the risk of side effects increases to the same degree.

Said block onset time is related above all to the diffusivity of the anesthetic, whereas from this standpoint the dose and its concentration are less relevant. In particular, the anesthetic is diffusible in non-ionized form and the non-ionized fraction is related to the intrinsic features of the drug and to the pH of the medium: if the latter is alkaline, diffusivity is higher. In this regard, studies on isolated nerve, conducted by using different agents blocking the nerve impulse, at constant extracellular pH, highlighted that non-ionized anesthetics, as well as ionisable tertiary amines are potentiated by sodium bicarbonate buffer. (Wong K, Strichartz GR, Raymond SA "On the mechanism of potentiation of local anaesthetics by bicarbonate buffer: drug structure-activity studies on isolated peripheral nerve", Anesth. Analg. 76,131, 1993).

A relevant experience in obstetrics, conducted on over 600 cases, has confirmed that the use of modified-pH mepivacaine reduces the latency time of these local anesthetics without side effects on the mother and on the fetus during delivery by C-section. (Varrassi G, Capogna D, Celleno G "Analgesia ed anestesia in ostetricia", Interservice Press 1991:132).

The reduction in latency time and the greater depth of the block produced are due to CO₂ diffusion through the nerve membrane, with the entailed diminution of the axoplasmatic pH. The low pH increases the rate and the degree of formation of the protonated active forms of the local anesthetic. In addition, the ionized local anesthetic does not diffuse easily through the membranes; therefore, the drug remains trapped inside the axoplasm, a situation known as "ion trap". Therefore, altering the solution by raising the pH increases the diffusion rate of the anesthetic through the nerve membrane (cathion trap).

Another interesting clinical data is that alkalized solutions are locally better tolerated with respect to unmodified ones (Martin AJ. "pH-adjustment and discomfort caused by the intradermal injection of lignocaine", Anaesthesia 1991 Mar, 46(3),242). This fact makes particularly useful these anesthetics in those regions where the pain caused by the infiltration of the solution (e.g. in day surgery and peripheral blocks) is felt to a greater extent.

The local anesthetics at issue, like e.g., mepivacaine, are currently available in phials. At the time of administration, the phial is broken and the anesthetic, for the reasons disclosed hereto, is more or less dissolved with 0.9% NaCl physiological solution and adjuvated with sodium bicarbonate (NaHCO₃). Therefore, such a solution is obtained extemporarily and manually, in a substantially empirical manner, generally obtaining a pH of the solution itself higher than the one associated to the sole anesthetic. However, given the risk of precipitation of the bicarbonate in the solution and the empirical modes of obtainment, the end pH is practically always lower than that theoretically obtainable with a perfectly saturated solution. In particular, to date the commercial preparations available in Italy have a weakly basic pH (e.g. equal to about 6.4 for the anesthetic Lidocaine, about 6.0 for Bupivacaine and about 6.2 for Mepivacaine).

Moreover, evidently the preparation modes expose the solution to the risk of environmental contamination, due also to the possibility that glass shards of the phials, dusts or keratins fall into the solution-containing container.

Addition of bicarbonate to the local anesthetics, besides from causing a modification of the pH, entails also an increase of the pCO₂ that performs an action synergic to that of the pH modification. However, this second action is lost when the addition of bicarbonate is carried out, as in the known art, in an open system and when the solution is not prepared at the time of blocking, since CO₂ disperses.

US 4,623,334 discloses an intravenous drug administration apparatus, which drug may contain lidocaine.

Hence, the technical problem underlying the present invention is to provide an alkalized local anesthetic in a suitable pharmaceutical form allowing to overcome the drawbacks mentioned hereto with reference to the known art.

Such a problem is solved by a pharmacological product according to claim 1.

The present invention provides several relevant advantages. The main advantage lies in that the in-bag packaging of the alkalized local anesthetic allows to attain an optimal, and in particular a saturated solution of anesthetic and alkalizing agent, and to preserve the properties of such a solution even for lengthy periods of time, at a 5-25 °C temperature, hence with no need of extemporary preparation.

Moreover, the anesthetic thus provided is stable at any concentration and has certain pH.

Other advantages, features and the operation modes of the present invention will be made apparent in the following detailed description of some embodiments thereof, given by way of example and not for limitative purposes. Reference will be made to the figures of the annexed drawings, wherein:
- Figure 1 shows a partially sectional front perspective view of a pharmacological product according to a first embodiment of the invention;
- Figure 1A shows a sectional view of the product of Figure 1 taken along line A-A of the latter;
- Figures 2 to 5 show each a respective perspective view of the product of Figure 1 during a clinical use thereof;
- Figure 6 shows a further perspective view of the product of Figure 1 during the opening of its pack prior of the clinical use thereof.

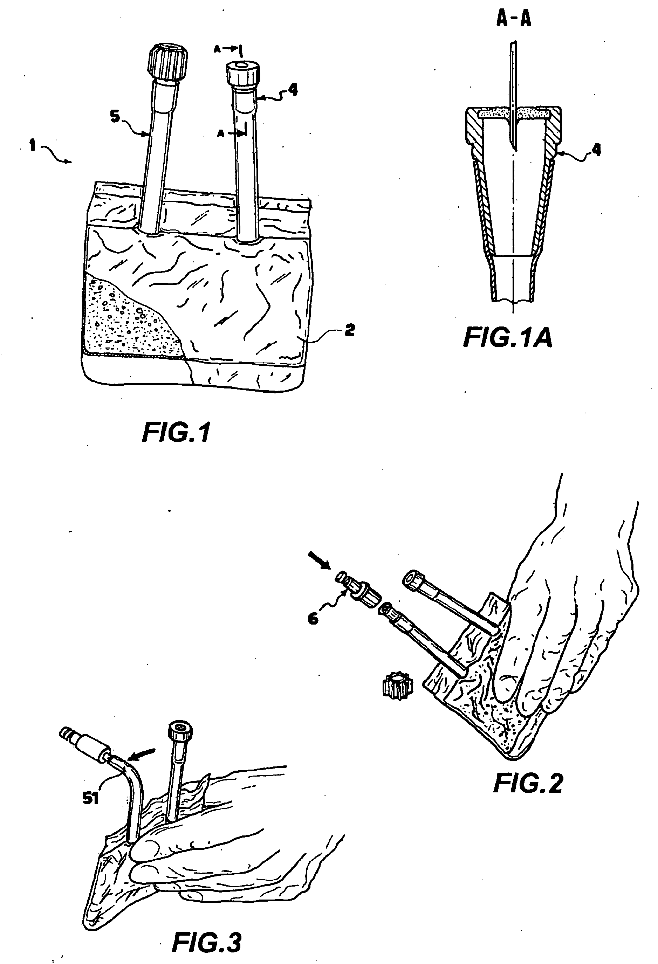

Referring to Figure 1, a pharmacological product according to a first embodiment of the invention is generally denoted by 1.

The product 1 comprises a substantially bag-shaped container body 2 of highly biocompatible material, receiving therein a pharmacological fluid 3 consisting in a solution of a local anesthetic with an alkalizing agent, in particular NaHC0₃.

In the present embodiment, the local anesthetic is mepivacaine hydrochloride and the product 1 contains alkalized mepivacaine at a 2% concentration. Always in the present embodiment, 1 ml of injectable solution contains:
- active principle: 20 mg mepivacaine hydrochloride, equal to 17.4 mg mepivacaine;
- excipients: 6 mg sodium chloride, sodium bicarbonate as needed to pH amendment, water for injectable preparations as needed to 1 ml.

Other embodiments may also have a concentration equal, e.g., to 0.08%, 0.12%, 0.25%, 1%, 1.5% or other, and different volumes (50, 100, 250, 500 ml).

The solution 3 is saturated, having a pH comprised in a range of about 8-8.5.

In general, the pH value of the saturated solution 3 depends on viscosity, surrounding environmental condition, and material of the container 2.

In any case, each bag complying with different concentrations and volumes was contrived, in the present invention, to make up for the needs of a sizeable part of the individual anesthesiological and surgical protocols. E.g., usually for a local infiltration of an inguinal hernia it is extemporarily prepared 50 ml of a solution containing 1% mepivacaine-HCl (hydrochloride), then a dose of 500mg HCl mepivacaine in 50ml, adjuvated with about 10 milliequivalents of NaHCO₃. Let us see the actual steps: the forms currently available in a hospital provide 10ml-phials of 2% mepivacaine. To produce the above solution, two phials should be broken up; furthermore, diluent (often 0.9% NaCl present in a hospital, rather than water for injectable preparations) and then milliequivalents of NaHCO₃ should be added to this distribution.

The above, besides from proving less than practical, is often produced in clinically unsuitable environments and exposes the staff in charge to the following main risks: accidental needle pricks, accidental cuts, preparation errors.

Another example is the production of an anesthetic solution useful for liposuction, better known under the name of Klein's solution.

This solution envisages a 0.08% concentration and a 500ml volume, then a dose of 400mg mepivacaine-HCl in 500ml H2O for injectable preparations or 0.9% NaCl physiological solution, plus the related milliequivalents of NaHCO₃.

To prepare it (usually in the operating room "steel beans") it is necessary to break up two 10ml-phials of 2% mepivacaine, add 480ml of 0.9% NaCl, then the phial/s of NaHCO₃.

In our case, also this solution may be industrially prepared according to protocol or with methods that the individual operator may provide in advance on prescription forms (Title 25, Para.4, Decree 178/91).

The container 2 may receive 20, 50, 100, 250 ml of solution, or other volumes.

Preferably, the container 2 is externally and internally sterile and see-through and made of a bilaminate material bearing an internal layer, i.e. into contact with the solution 3, of polypropylene and an external layer of polyvinyl chloride (PVC). Thus, the container 2 provides to the touch the plastic quality of PVC and keeps the optimal properties of compatibility with the solution 3 of polypropylene.

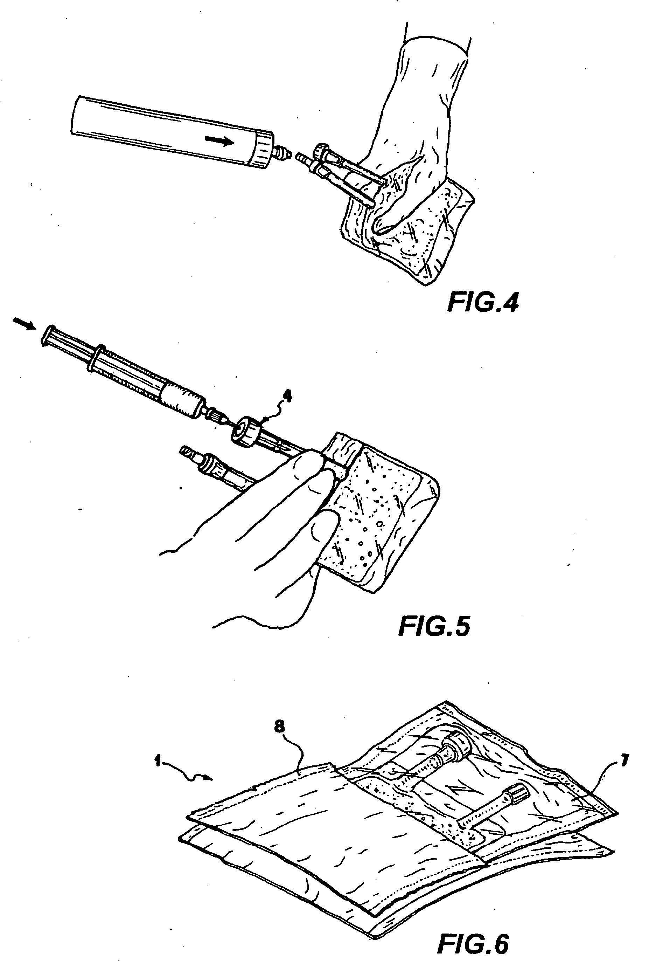

The container 2 has a self-sealing element 4, per se known and anyhow shown in greater detail in Figure 1A, apt to allow the repeated extraction of the solution 3 by a needle or the like. Such operation modes are illustrated in greater detail in Figure 5.

Moreover, the container 2 has an inserting or connecting element 5 for a multiple-collection connector 6, it also per se known and anyhow shown in greater detail in Figures 2, 3 and 4. The connecting element 5 is in the form of a channel making a seat for the connector 6 and connecting the internal housing of the container 2 itself to the outside. Such a channel has a transversal septum 51 apt to be torn at the first use, by a mere folding of, or pressure onto the channel-shaped seat, as shown in Figure 3.

Referring to Figure 6, at the packaging of the product 1, the container 2 is completely enclosed in a preferably sterile first external wrapper 7, which is removed at the time of use of the product 1 itself. For this purpose, the wrapper 7 may have a known-type preferential tearing region.

Preferably, the external wrapper 7 is see-through, so as to allow the view of the container 2 and of the solution 3, and made of polypropylene.

Moreover, the product 1 provides a further external wrapper 8, which encloses the first wrapper 7 and is it also removable at the time of using the product 1 itself. The second wrapper 8 is apt to shield the solution 3 from light and environmental contaminations and is preferably made of aluminium. It also may have a preferential tearing region.

Of course, variant embodiments could provide the use of only one of the two above-described wrappers 7, 8. However, the provision of a double wrapper shielding the actual container 2 from a contact with the environment assures an optimal preservation of the solution 3 and its use under perfectly sterile conditions.

The product 1 is normally provided with the connector 6 being separate, so as to allow autoclave sterilization of the container 2 and of the wrappers 7 and 8.

Concerning the preparation modes of the solution 3, these may provide a partitioning of the overall amount of local anesthetic to be alkalized (the so-called "bulk") and the gradual and separated addition of sodium bicarbonate to each of the partitioned subquantities.

By now, it will have been better appreciated that the invention allows a simpler, more effective and safer management of local-regional anesthesia and continuous infiltration. Experimental tests conducted by the Inventors highlighted that the suitably alkalized anesthetic solutions of the invention yield an increase of the amount of drug in the form of undissociated base, increasing the diffusion rate through the nerve membrane and sheath, with a more rapid onset of the anesthesia, and moreover making available a greater amount at the level of the action site. In addition, these tests highlighted the improved performances of the solution with regard to absence of systemic toxicity and potentiation of the anesthesia. Ultimately, the latency time of the solution disappears and the duration increases of the 40-50%.

The present invention has hereto been described with reference to preferred embodiments thereof. It is understood that other embodiments might exist, all referable to the same inventive kernel, and all comprised within the protective scope of the claims hereinafter.

## Claims

1. A pharmacological product (1) comprising:
- a container (2) substantially bag-shaped and apt to receive a pharmacological fluid,
**characterised in that** it further comprises
- a solution (3) containing an alkalized local anesthetic received in said container (2).

2. The product (1) according to claim 1, wherein said local anesthetic is selected from a group comprising mepivacaine, bupivacaine, lidocaine.

3. The product (1) according to claim 1 or 2, wherein said solution (3) has a pH comprised in a range of about 8.0-8.5.

4. The product (1) according to any one of the preceding claims, wherein said container (2) is transparent.

5. The product (1) according to any one of the preceding claims, wherein said container (2) is made of a bilaminate material.

6. The product (1) according to the preceding claim, wherein said container (2) has an internal layer, into contact with said solution (3), of polypropylene.

7. The product (1) according to claim 5 or 6, wherein said container (2) has an external layer of PVC.

8. The product (1) according to any one of the preceding claims, wherein said container (2) is sterile.

9. The product (1) according to any one of the preceding claims, wherein said container (2) comprises a self-sealing element (4) apt to allow the repeated extraction of said solution (3) by a needle.

10. The product (1) according to any one of the preceding claims, wherein said container (2) comprises a connecting element (5) for a multiple-collection connector (6).

11. The product (1) according to the preceding claim, wherein said connecting element (5) has a transversal septum (51) apt to be torn at the first use thereof.

12. The product (1) according to any one of the preceding claims, further comprising a multiple-collection connector (6) for the extraction of said solution (3).

13. The product (1) according to any one of the preceding claims, comprising an external wrapper (7; 8) that completely shields said container (2) from contact with the environment and that is removable at the time of use.

14. The product (1) according to the preceding claim, wherein said external wrapper (7) is sterile.

15. The product (1) according to claim 13 or 14, wherein said external wrapper (7) is made of polypropylene.

16. The product (1) according to any one of the claims 13 to 15, wherein said external wrapper (8) is apt to shield said container (2) from light.

17. The product (1) according to any one of the claims 13 to 16, wherein said external wrapper (8) is made of aluminium.

18. The product (1) according to any one of the claims 13 to 15, wherein said external wrapper (7) is transparent.

19. The product (1) according to any one of the preceding claims, comprising a first wrapper (7) enclosing said container (2) and a second wrapper (8) enclosing said first wrapper (7) and shielding it completely from contact with the environment, both of said wrappers (7, 8) being removable at the time of use.

20. The product (1) according to the preceding claim, wherein said first wrapper (7) is transparent.

21. The product (1) according to claim 19 or 20, wherein said first wrapper (7) is sterile.

22. The product (1) according to any one of the claims 19 to 21, wherein said second wrapper (8) is apt to shield said container (2) from light.

23. The product (1) according to any one of the claims 19 to 22, wherein said second wrapper (8) is made of aluminium.

## Patentansprüche

1. Pharmakologisches Erzeugnis, das umfasst:
- einen Behälter (2), der im Wesentlichen beutelförmig und in der Lage ist, ein pharmakologisches Fluid aufzunehmen"
**dadurch gekennzeichnet, dass** es weiter umfasst
- eine Lösung (3) die ein alkalisiertes Lokalanästhetikum enthält und in dem Behälter (2) aufgenommen ist.

2. Erzeugnis (1) nach Anspruch 1, wobei das Lokalanästhetikum aus einer Gruppe ausgewählt ist, die Mepivacain, Bupivacain, Lidocain umfasst.

3. Erzeugnis (1) nach Anspruch 1 oder 2, wobei die Lösung (3) einen pH-Wert hat, der in einem Bereich von etwa 8,0 - 8,5 liegt.

4. Erzeugnis (1) nach einem der vorhergehenden Ansprüche, wobei der Behälter (2) transparent ist.

5. Erzeugnis (1) nach einem der vorhergehenden Ansprüche, wobei der Behälter (2) aus einem Bilaminat-Material hergestellt ist.

6. Erzeugnis (1) nach dem vorhergehenden Anspruch, wobei der Behälter (2) eine innere Schicht aus Polypropylen in Kontakt mit der Lösung (3) aufweist.

7. Erzeugnis (1) nach Anspruch 5 oder 6, wobei der Behälter (2) eine äußere Schicht aus PVC aufweist.

8. Erzeugnis (1) nach einem der vorhergehenden Ansprüche, wobei dem der Behälter (2) steril ist.

9. Erzeugnis (1) nach einem der vorhergehenden Ansprüche, wobei der Behälter (2) ein selbstabdichtendes Element (4) für die wiederholte Entnahme der Lösung (3) durch eine Nadel aufweist.

10. Erzeugnis (1) nach einem der vorhergehenden Ansprüche, wobei der Behälter (2) ein Verbindungselement (5) für einen Mehrfachsammelverbinder (6) aufweist.

11. Erzeugnis (1) nach dem vorhergehenden Anspruch, wobei das Verbindungselement (5) eine transversale Trennwand (51) aufweist, die bei dem ersten Gebrauch desselben zerreißbar ist.

12. Erzeugnis (1) nach einem der vorhergehenden Ansprüche, weiter mit einem Mehrfachsammelverbinder (6) für die Entnahme der Lösung (3).

13. Erzeugnis (1) nach einem der vorhergehenden Ansprüche, mit einer äußeren Hülle (7; 8), die den Behälter (2) vor einem Kontakt mit der Umgebung vollständig abschirmt und die zur Zeit des Gebrauches entfernbar ist.

14. Erzeugnis (1) nach dem vorhergehenden Anspruch, wobei die äußere Hülle (7) steril ist.

15. Erzeugnis (1) nach Anspruch 13 oder 14, wobei die äußere Hülle (7) aus Polypropylen besteht.

16. Erzeugnis (1) nach einem der Ansprüche 13 bis 15, wobei die äußere Hülle (8) in der Lage ist, den Behälter (2) vor Licht abzuschirmen.

17. Erzeugnis (1) nach einem der Ansprüche 13 bis 16, wobei die äußere Hülle (8) aus Aluminium besteht.

18. Erzeugnis (1) nach einem der Ansprüche 13 bis 15, wobei die äußere Hülle (7) transparent ist.

19. Erzeugnis (1) nach einem der vorhergehenden Ansprüche, mit einer ersten Hülle (7), die den Behälter (2) umschließt, und einer zweiten Hülle (8), die die erste Hülle (7) umschließt und diese vollständig vor einem Kontakt mit der Umgebung abschirmt, wobei beide Hüllen (7; 8) zur Zeit des Gebrauches entfernbar sind.

20. Erzeugnis (1) nach dem vorhergehenden Anspruch, wobei die erste Hülle (7) transparent ist.

21. Erzeugnis (1) nach Anspruch 19 oder 20, bei dem die erste Hülle (7) steril ist.

22. Erzeugnis (1) nach einem der Ansprüche 19 bis 21, bei dem die zweite Hülle (8) in der Lage ist, den Behälter (2) vor Licht abzuschirmen.

23. Erzeugnis (1) nach einem der Ansprüche 19 bis 22, wobei die zweite Hülle (8) aus Aluminium besteht.

## Revendications

1. Produit pharmaceutique (1) comprenant :
- un récipient (2) qui a sensiblement la forme d'une poche et qui est apte à recevoir un fluide pharmacologique ;
et **caractérisé en ce qu'**il comprend en outre :
- une solution (3) contenant un anesthésique local alcalinisé, contenue dans ledit récipient (2).

2. Produit (1) conforme à la revendication 1, pour lequel ledit anesthésique local est choisi dans l'ensemble formé par les mépivacaïne, bupivacaïne et lidocaïne.

3. Produit (1) conforme à la revendication 1 ou 2, dans lequel ladite solution (3) présente un pH qui se situe dans l'intervalle allant à peu près de 8,0 à 8,5.

4. Produit (1) conforme à l'une des revendications précédentes, dans lequel ledit récipient (2) est transparent.

5. Produit (1) conforme à l'une des revendications précédentes, dans lequel ledit récipient (2) est fait d'un matériau stratifié bicouche.

6. Produit (1) conforme à la revendication précédente, dans lequel ledit récipient (2) comporte une couche interne, au contact de ladite solution (3), qui est en polypropylène.

7. Produit (1) conforme à la revendication 5 ou 6, dans lequel ledit récipient (2) comporte une couche externe qui est en poly(chlorure de vinyle).

8. Produit (1) conforme à l'une des revendications précédentes, dans lequel ledit récipient (2) est stérile.

9. Produit (1) conforme à l'une des revendications précédentes, dans lequel ledit récipient (2) comporte un élément auto-scellant (4) qui est adapté pour permettre d'extraire de ladite solution (3) de manière répétitive à l'aide d'une aiguille.

10. Produit (1) conforme à l'une des revendications précédentes, dans lequel ledit récipient (2) comporte un élément de raccordement (5) adapté pour un raccord (6) de collecte multiple.

11. Produit (1) conforme à la revendication précédente, dans lequel ledit élément de raccordement (5) comporte un septum transversal qui peut être déchiré lorsqu'on se sert pour la première fois du produit.

12. Produit (1) conforme à l'une des revendications précédentes, qui comprend en outre un raccord (6) de collecte multiple pour l'extraction de ladite solution (3).

13. Produit (1) conforme à l'une des revendications précédentes, qui comprend un emballage externe (7 ; 8) qui protège parfaitement ledit récipient (2) de tout contact avec le milieu environnant et que l'on peut retirer au moment d'utiliser le produit.

14. Produit (1) conforme à la revendication précédente, dans lequel ledit emballage externe (7) est stérile.

15. Produit (1) conforme à la revendication 13 ou 14, dans lequel ledit emballage externe (7) est en polypropylène.

16. Produit (1) conforme à l'une des revendications 13 à 15, dans lequel ledit emballage externe (8) est adapté pour abriter ledit récipient (2) de la lumière.

17. Produit (1) conforme à l'une des revendications 13 à 16, dans lequel ledit emballage externe (8) est en aluminium.

18. Produit (1) conforme à l'une des revendications 13 à 15, dans lequel ledit emballage externe (7) est transparent.

19. Produit (1) conforme à l'une des revendications précédentes, qui comprend un premier emballage (7), qui renferme ledit récipient (2), et un deuxième emballage (8), qui renferme ledit premier emballage (7) et le protège parfaitement de tout contact avec le milieu environnant, ces deux emballages (7, 8) pouvant être retirés au moment d'utiliser le produit.

20. Produit (1) conforme à la revendication précédente, dans lequel ledit premier emballage (7) est transparent.

21. Produit (1) conforme à la revendication 19 ou 20, dans lequel ledit premier emballage (7) est stérile.

22. Produit (1) conforme à l'une des revendications 19 à 21, dans lequel ledit deuxième emballage (8) est adapté pour abriter ledit récipient (2) de la lumière.

23. Produit (1) conforme à l'une des revendications 19 à 22, dans lequel ledit deuxième emballage (8) est en aluminium.
